# EUROPÄISCHE PATENTSCHRIFT

(11) **EP 3 281 930 B1**
(45) Veröffentlichungstag und Bekanntmachung des Hinweises auf die Patenterteilung: **30.09.2020**
(21) Anmeldenummer: 17001345.2
(22) Anmeldetag: 07.08.2017
(51) Int. Cl.: A01K 15/02, G01N 33/00, C06B 23/00

(54) **GERUCHSPROBE**
ODOUR SAMPLE
ÉCHANTILLON ODORANT

(30) Priorität: 12.08.2016 DE 102016009872
(43) Veröffentlichungstag der Anmeldung: 14.02.2018
(73) Patentinhaber: Diehl Defence GmbH & Co. KG, 88662 ÜBERLINGEN (DE)
(72) Erfinder: Hahma, Arno, DE - 91239 Henfenfeld (DE); Pham-Schönwetter, Oliver, DE - 91207 Lauf (DE)
(74) Vertreter: Diehl Patentabteilung

(56) Entgegenhaltungen:
- EP-A1- 2 698 361
- EP-A2- 2 698 359
- WO-A2-2004/035018
- DE-A1-102009 029 787
- US-A1- 2008 251 169

## Beschreibung

Die Erfindung betrifft eine Geruchsprobe für Sprengstoffspürhunde, die eine Lösung eines Sprengstoffs in einer ionischen Flüssigkeit umfasst.

Aus der DE 10 2009 029 787 A1 ist es bekannt, als Duftquelle zum Training von Sprengstoffspürhunden eine Lösung einer neutralen ionischen Flüssigkeit mit einer detektierbaren Menge eines peroxidischen Sprengstoffs zu verwenden. Bei dem peroxidischen Sprengstoff kann es sich beispielsweise um Triacetontriperoxid (TATP) oder Hexamethylentriperoxiddiamin (HMTD) handeln. Durch das Lösen des Sprengstoffs in dem ionischen Lösungsmittel ergibt sich eine stabile und leicht handhabbare Form des jeweiligen Sprengstoffs. Die mechanische und thermische Empfindlichkeit des Sprengstoffs ist durch die Lösung in der ionischen Flüssigkeit deutlich reduziert, so dass die Lösung dadurch in konventionellen Laboratorien mit üblicher Ausrüstung einfach zu handhaben ist. Das Lösen des peroxidischen Sprengstoffs in Kombination mit einer reduktiv wirkenden Komponente in der ionischen Flüssigkeit deaktiviert den Sprengstoff dauerhaft und kann zur Entschärfung genutzt werden. Durch reduktiv wirkende ionische Flüssigkeiten kann ein phlegmatisierender Abbau der peroxidischen Sprengstoffe erfolgen. Weitere Sprengstoff enthaltende Zusammensetzungen werden in US 2008/251169, EP 2 698 359, EP 2 698 361 und DE 10 2009 029787 offenbart.

Aufgabe der vorliegenden Erfindung ist es, eine alternative Duftquelle zum Training von Sprengstoffspürhunden, ein Verfahren zu deren Herstellung und eine Verwendung dieser Duftquelle bereitzustellen.

Die Aufgabe wird durch die Merkmale der Patentansprüche 1, 11 und 12 gelöst. Zweckmäßige Ausgestaltungen ergeben sich aus den Merkmalen der Patentansprüche 2 bis 10. Erfindungsgemäß ist eine Geruchsprobe für Sprengstoffspürhunde vorgesehen, umfassend eine Lösung des Sprengstoffs in einer ionischen Flüssigkeit, wobei der Sprengstoff ein nicht-peroxidischer Sprengstoff ist, und wobei der Sprengstoff in der ionischen Flüssigkeit in einer Konzentration von höchstens 15 Gew.-% vorliegt. Die Erfinder der erfindungsgemäßen Geruchsprobe haben überraschenderweise festgestellt, dass sich auch nicht-peroxidische Sprengstoffe, wie beispielsweise Hexogen (RDX), Oktogen (HMX), Nitropenta (PETN), Tetryl oder Trinitrotoluol (TNT), gut und auch in hoher Konzentration in ionischen Flüssigkeiten lösen lassen. Durch das Auflösen in der ionischen Flüssigkeit wird der Sprengstoff vollständig gegenüber Reibung, Schlag, Stoß, Schock, Brand und jeglicher anderer Belastung desensibilisiert. Die Lösung ist nicht explosiv und fällt damit auch nicht unter eine Explosivstoffe umfassende Gefahrenklasse, sondern nur noch unter eine brennbare Flüssigkeiten umfassende Gefahrenklasse, wobei ionische Flüssigkeiten an sich nicht brennbar sind. Die erfindungsgemäße Geruchsprobe fällt somit nicht unter das deutsche Sprengstoffgesetz. Sie kann ohne Probleme als Chemikalie transportiert und gehandhabt werden. Dies gilt selbst dann, wenn der nicht-peroxidische Sprengstoff in der ionischen Flüssigkeit in verhältnismäßig hoher Konzentration gelöst ist. Zur hohen Sicherheit der erfindungsgemäßen Geruchsprobe trägt auch bei, dass der darin enthaltene Sprengstoff nur mit hohem technischen Aufwand, z. B. einer Chromatographie, von der ionischen Flüssigkeit getrennt werden kann. Die so erhaltbare Menge an Sprengstoff ist gering. Ein Missbrauch der erfindungsgemäßen Geruchsprobe zur Gewinnung einer relevanten Menge an Sprengstoff ist dadurch nahezu ausgeschlossen.

Die erfindungsgemäße Geruchsprobe kann, wenn sie z. B. in einem geeigneten, zuschraubbaren Behältnis gelagert wird, für mindestens 1 Jahr zum Training von Sprengstoffspürhunden verwendet werden. Die ionische Flüssigkeit an sich hat keinen Eigengeruch, da der Dampfdruck ionischer Flüssigkeiten vernachlässigbar gering ist. Die Sprengstoffspürhunde können dadurch den reinen Sprengstoffgeruch wahrnehmen.

Bei der ionischen Flüssigkeit kann es sich um eine lipophile ionische Flüssigkeit handeln. Diese ist zum Lösen unpolarer nicht-peroxidischer Sprengstoffe besonders gut geeignet. Ein weiterer Vorteil der lipophilen ionischen Flüssigkeit besteht darin, dass sie verhältnismäßig wenig Wasser aus der Umgebung aufnimmt. Wasser kann die Haltbarkeit der erfindungsgemäßen Geruchsprobe, insbesondere bei einem hydrolyseempfindlichen nicht-peroxidischen Sprengstoff, herabsetzen. Durch aufgenommenes Wasser kann eine, insbesondere hydrolytische, Zersetzung des Sprengstoffs und dadurch auch eine Veränderung des Geruchs der Geruchsprobe erfolgen. Die lipophile ionische Flüssigkeit kann lipophile Anionen enthalten.

Die Eigenschaften der ionischen Flüssigkeit werden sowohl von den die ionische Flüssigkeit bildenden Anionen als auch von den die ionische Flüssigkeit bildenden Kationen beeinflusst. Dadurch ist durch die Wahl der Kombination der Anionen und Kationen eine Anpassung der ionischen Flüssigkeit an das Löseverhalten des jeweiligen Sprengstoffs möglich.

Es kann eine ionische Flüssigkeit mit verhältnismäßig geringer Viskosität gewählt werden. Dies ist besonders gut zum Tränken eines saugfähigen Trägermaterials, wie beispielsweise Kieselgur, geeignet.

Bei den in der ionischen Flüssigkeit enthaltenen Anionen kann es sich um Anionen handeln, die aus einer Gruppe ausgewählt sind, die Tetrafluorborate, Triflimide, Perfluoralkylsulfate, Alkylsulfonate, Dicyandiamide, Alkylsulfate, Arylsulfonate, Perfluoralkylsulfonate, bis-Perfluoralkylsulfonimide, Acetate, Alkylcarboxylate, Thiocyanate, Isocyanate, Isothiocyanate, Thiosulfate, Borate, Borhydride, Phosphate, Nitrate, Perchlorate und Halogenide, insbesondere lodide, Bromide, Chloride und Fluoride, umfasst.

Die ionische Flüssigkeit kann Kationen enthalten, die aus einer Gruppe ausgewählt sind, die N-alkylsubstituierte Stickstoffheterozyklusionen, insbesondere N-Alkylpyridinium-, N-Alkylimidazolium- und N,N-Dialkylimidazoliumionen, quartäre Ammoniumionen und Phosphoniumionen, umfasst. Gut geeignet sind insbesondere N,N-Dialkylimidazolium- und N-Alkylpyridiniumionen.

Die ionische Flüssigkeit kann aus einer Gruppe ausgewählt sein, die 1-Ethyl-3-methyl-imidazolium-ethylsulfat, 1-Ethyl-3-methylimidazolium-bis(trifluormethansulfonimid), 1-Butyl-3-methylimidazolium-bis(trifluormethansulfonimid), 1-Hexyl-3-methylimidazolium-bis(trifluormethansulfonimid), 1-Ethyl-3-methylimidazolium-thiocyanat, 1-Butyl-3-methylimidazolium-thiocyanat, 1-Ethyl-3-methylimidazolium-tetrafluorborat, N-(n-Hexyl)pyridinium-tetrafluorborat, N-(n-Hexyl)pyridinium-bis(trifluormethansulfonimid), N-(n-Butyl)-3-methylpyridinium-tetrafluorborat, N-(n-Butyl)-4-methylpydirdinium-tetrafluorborat, N-Methylpyrrolidin-Zink-Borhydrid, 1-Allyl-3-n-butyl-imidazolium-borhydrid, 1,3-Diallylimidazolium-borhydrid, 1,3-Di-(n-octyl)-imidazolium-borhydrid und 1,3-Di-(n-butyl)-imidazolium-borhydrid umfasst.

Bei einer Ausführungsform der Erfindung wirkt die ionische Flüssigkeit selbst gegenüber dem Sprengstoff reduzierend oder die ionische Flüssigkeit enthält ein den Sprengstoff in der ionischen Flüssigkeit reduzierendes Reduktionsmittel. Durch eine Reduktion des Sprengstoffs wird dieser so zerstört, dass auch eine theoretisch mit hohem Aufwand, beispielsweise mittels Chromatographie, mögliche Separation des Sprengstoffs von der ionischen Flüssigkeit keinen funktionierenden Sprengstoff mehr liefern kann. Dadurch wird die Möglichkeit eines Missbrauchs der erfindungsgemäßen Geruchsprobe zur Sprengstoffgewinnung nahezu unmöglich gemacht. Bei dem Reduktionsmittel kann es sich beispielsweise um einen Zucker, ein Sulfit, Dithionit, Thiosulfat, Hydrazin, Boran, Phosphin, ein Hydrid, Zink, ein Siloxan oder ein Silan handeln. Der Zucker kann Glukose, Puderzucker, Fructose, Galactose, Maltose oder Lactose sein. Bei dem Hydrid kann es sich um ein Metallhydrid, wie beispielsweise Lithiumaluminiumhydrid oder Borhydrid, handeln.

Bei der selbst gegenüber dem Sprengstoff reduzierend wirkenden ionischen Flüssigkeit kann es sich um ein Thiocyanat, insbesondere 1-Ethyl-3-methylimidazolium-thiocyanat oder 1-Butyl-3-methylimidazolium-thiocyanat, oder ein Borhydrid, insbesondere N-Methylpyrrolidin-Zink-Borhydrid, 1-Allyl-3-n-butyl-imidazolium-borhydrid, 1,3-Diallylimidazolium-borhydrid, 1,3-Di-(n-octyl)-imidazolium-borhydrid oder 1,3-Di-(n-butyl)-imidazolium-borhydrid, handeln.

Eine hohe Sicherheit im Hinblick auf eine Explosionsgefahr bietet eine erfindungsgemäße Geruchsprobe, bei welcher der Sprengstoff in der ionischen Flüssigkeit in einer Konzentration von höchstens 15 Gew.-%, insbesondere höchstens 12,5 Gew.-%, insbesondere höchstens 10 Gew.-%, vorliegt.

Damit die Geruchsprobe möglichst lange hält und ein möglichst geringes Volumen davon als Geruchsprobe für Sprengstoffspürhunde ausreichend ist, sollte der Sprengstoff in der ionischen Flüssigkeit in einer Konzentration von mindestens 1 Gew.-%, insbesondere mindestens 2,5 Gew.-%, insbesondere mindestens 5 Gew.-%, insbesondere mindestens 7,5 Gew.-%, insbesondere mindestens 9 Gew.-%, vorliegen. Die Erfindung betrifft weiterhin ein Verfahren zur Herstellung der erfindungsgemäßen Geruchsprobe, wobei der nicht-peroxidische Sprengstoff in der ionischen Flüssigkeit aufgelöst wird.

Die Erfindung betrifft darüber hinaus die Verwendung der erfindungsgemäßen Geruchsprobe als Duftquelle, insbesondere für Testmessungen für die Kalibrierung von Detektoren und zum Training von Sprengstoffspürhunden oder von anderen zum Aufspüren von Sprengstoff geeigneten Tieren, insbesondere Ratten.

Nachfolgend wird die Erfindung anhand von Ausführungsbeispielen näher erläutert. Es zeigen:

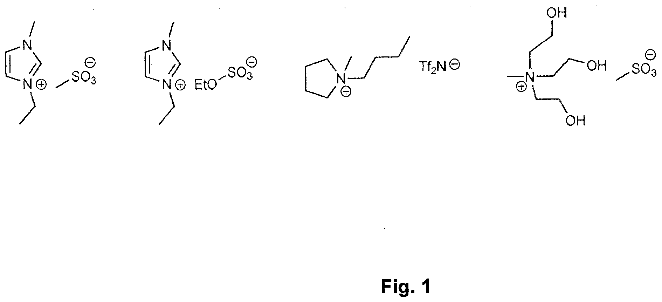

- Fig. 1: Strukturformeln von Beispielen ionischer Flüssigkeiten,
- Fig. 2: ein 1H-NMR-Spektrum von 1-Ethyl-3-methyl-imidazolium-ethylsulfat,
- Fig. 3: ein 1H-NMR-Spektrum von TNT,
- Fig. 4: ein 1H-NMR-Spektrum von 10 Gew.-% TNT in 1-Ethyl-3-methyl-imidazolium-ethylsulfat,
- Fig. 5: ein Headspace-Gaschromatographie-Massenspektrum des Gasraums in einem leeren Kolben,
- Fig. 6: ein Headspace-Gaschromatographie-Massenspektrum des Gasraums in einem 1-Ethyl-3-methyl-imidazolium-ethylsulfat enthaltenden Kolben und
- Fig. 7: ein Headspace-Gaschromatographie-Massenspektrum des Gasraums in einem 10 Gew.-% TNT in 1-Ethyl-3-methyl-imidazolium-ethylsulfat enthaltenden Kolben.

Eine 500 ml Glasflasche aus dunklem Glas, welche mittels eines Verschlusses mit Teflondichtung dicht verschlossen werden kann, wird mit einem Magnetrührkern versehen und mit 270 g 1-Ethyl-3-methyl-imidazolium-ethylsulfat befüllt. Unter Rühren werden portionsweise vorsichtig 30 g TNT zugegeben. Die Glasflasche wird mittels des Verschlusses verschlossen und für mindestens 12 Stunden mittels des Magnetrührkerns und einem Magnetrührer bei 350 Umdrehungen pro Minute gerührt, bis sich das TNT in der ionischen Flüssigkeit vollständig aufgelöst hat. Das Resultat ist eine 10 Gew.-% TNT enthaltende Lösung.

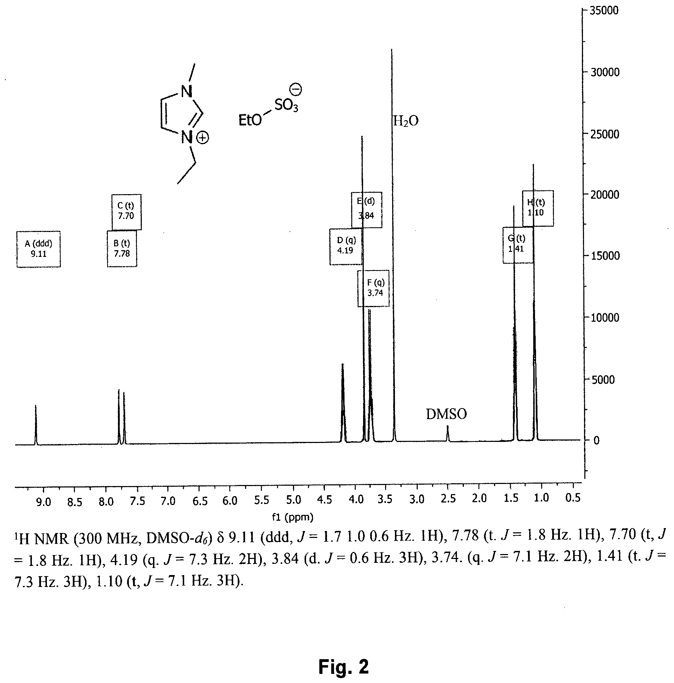

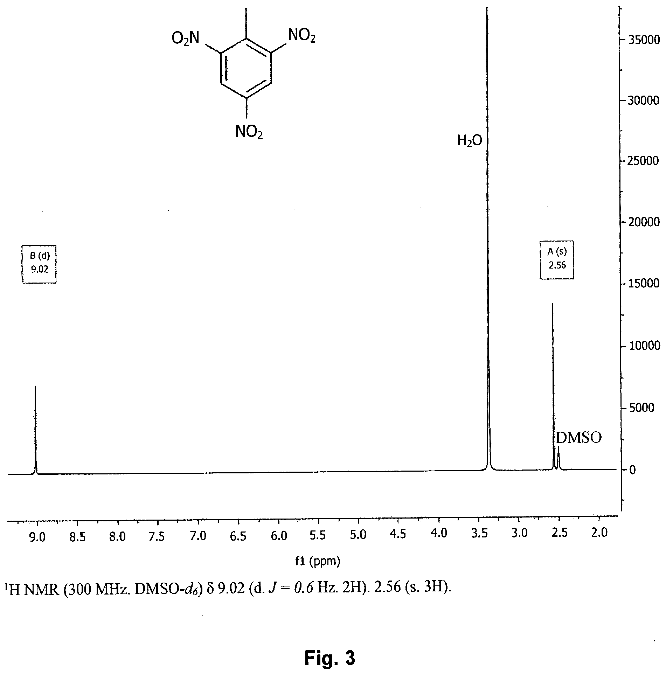

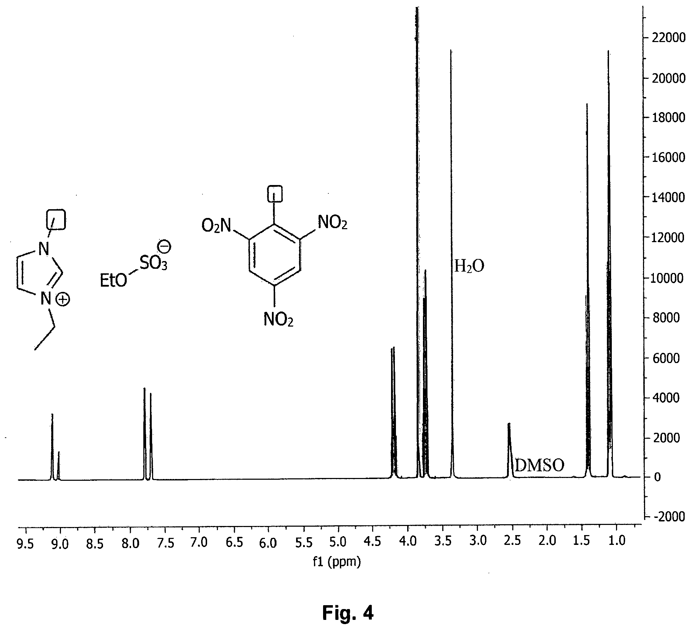

Ein Vergleich der 1H-NMR-Spektren von 1-Ethyl-3-methyl-imidazolium-ethylsulfat gemäß Fig. 2, TNT gemäß Fig. 3 und der wie oben beschrieben hergestellten TNT-Lösung gemäß Fig. 4 zeigt eindeutig das Vorhandensein von TNT in der ionischen Flüssigkeit. Der in den Spektren enthaltene Peak von DMSO resultiert aus einer Verunreinigung des als Lösungsmittel für die Spektren eingesetzten deuterierten DMSOs mit nicht deuteriertem DMSO.

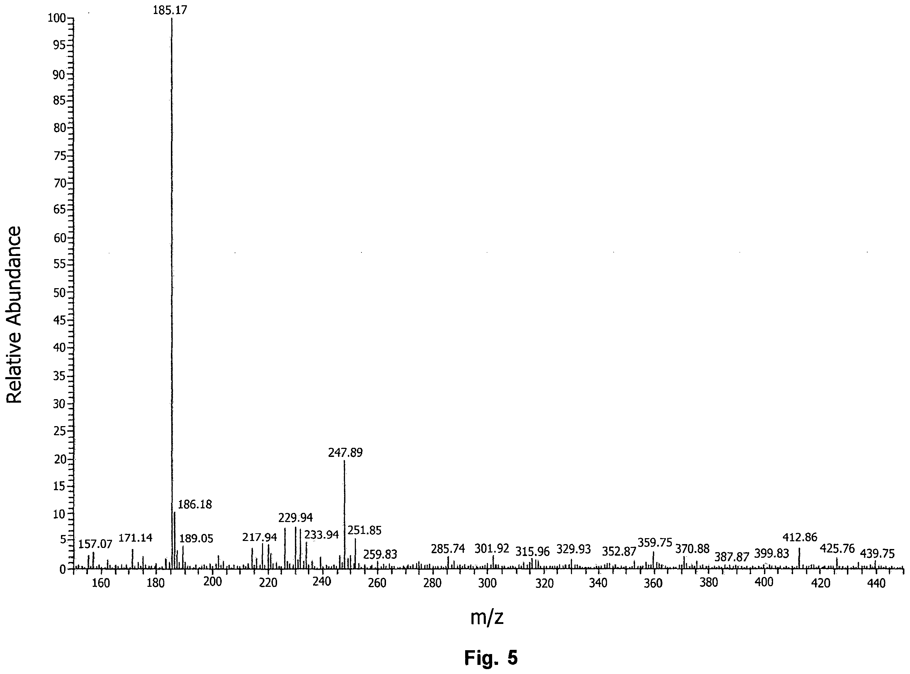

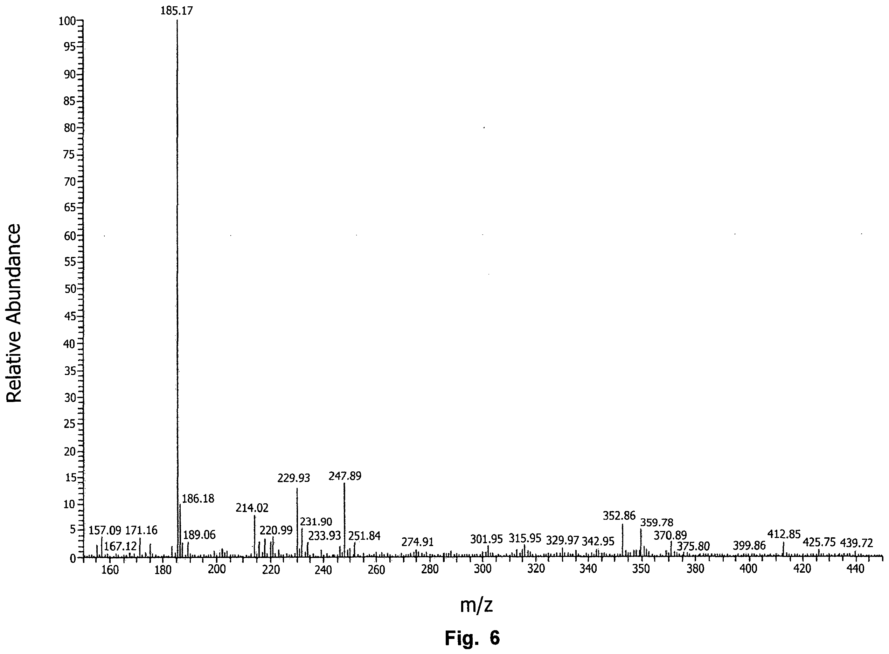

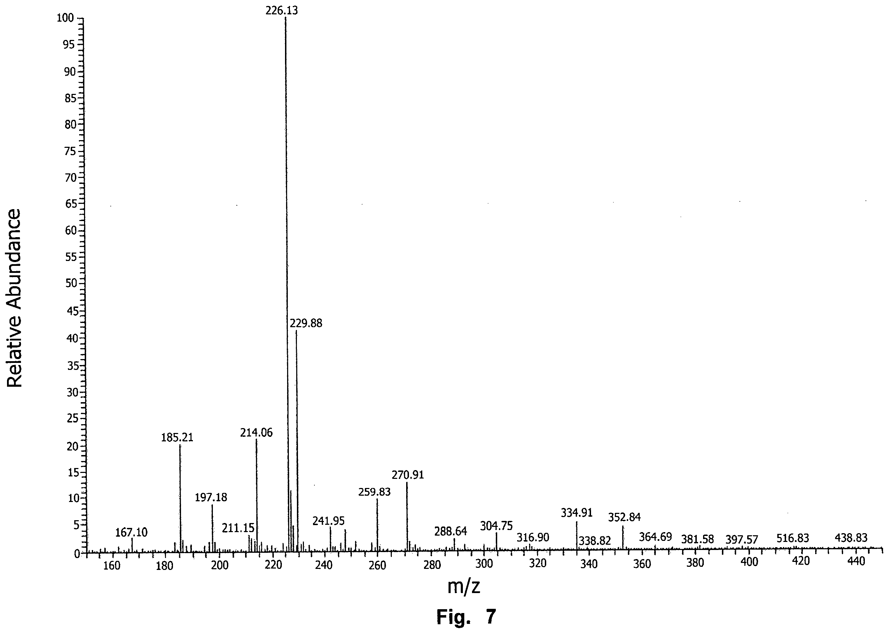

Aus dem Vergleich von Fig. 6 mit Fig. 5 ist ersichtlich, dass sich die im Massenspektrum der Gasphase über der ionischen Flüssigkeit nachweisbaren Substanzen nicht von den Substanzen unterscheiden, die sich im Massenspektrum des Gasraums im leeren Kolben nachweisen lassen. Im Gegensatz dazu zeigt das Massenspektrum der Gasphase über der 10 Gew.-% TNT enthaltenden ionischen Flüssigkeit gemäß Fig. 7 deutlich das Vorhandensein von TNT. Dies zeigt, dass die ionische Flüssigkeit keinen Einfluss auf die Stoffe in der Gasphase über der Lösung des Sprengstoffs in der ionischen Flüssigkeit hat und der Sprengstoff in dieser Gasphase nachweisbar und somit auch für einen Sprengstoffspürhund erschnüffelbar ist.

## Patentansprüche

1. Geruchsprobe für Sprengstoffspürhunde, umfassend eine Lösung eines Sprengstoffs in einer ionischen Flüssigkeit,
wobei der Sprengstoff ein nicht-peroxidischer Sprengstoff ist, und
wobei der Sprengstoff in der ionischen Flüssigkeit in einer Konzentration von höchstens 15 Gew.-% vorliegt.

2. Geruchsprobe nach Anspruch 1,
**dadurch gekennzeichnet,**
**dass** der nicht-peroxidische Sprengstoff Hexogen (RDX), Oktogen (HMX), Nitropenta (PETN), Tetryl oder Trinitrotoluol (TNT) ist.

3. Geruchsprobe nach einem der vorhergehenden Ansprüche,
**dadurch gekennzeichnet,**
**dass** die ionische Flüssigkeit Anionen enthält, die aus einer Gruppe ausgewählt sind, die Tetrafluorborate, Triflimide, Perfluoralkylsulfate, Alkylsulfonate, Dicyandiamide, Alkylsulfate, Arylsulfonate, Perfluoralkylsulfonate, bis-Perfluoralkylsulfonimide, Acetate, Alkylcarboxylate, Thiocyanate, Isocyanate, Isothiocyanate, Thiosulfate, Borhydride, Borate, Phosphate, Nitrate, Perchlorate und Halogenide, insbesondere lodide, Bromide, Chloride und Fluoride, umfasst.

4. Geruchsprobe nach einem der vorhergehenden Ansprüche,
**dadurch gekennzeichnet,**
**dass** die ionische Flüssigkeit Kationen enthält, die aus einer Gruppe ausgewählt sind, die N-alkylsubstituierte Stickstoffheterozyklusionen, insbesondere N-Alkylpyridinium-, N-Alkylimidazolium- und N,N-Dialkylimidazoliumionen, quartäre Ammoniumionen und Phosphoniumionen, umfasst.

5. Geruchsprobe nach einem der vorhergehenden Ansprüche,
**dadurch gekennzeichnet,**
**dass** die ionische Flüssigkeit aus einer Gruppe ausgewählt ist, die
1-Ethyl-3-methyl-imidazolium-ethylsulfat, 1-Ethyl-3-methylimidazolium-bis(trifluormethansulfonamid), 1-Butyl-3-methylimidazolium-bis(trifluormethansulfonimid), 1-Hexyl-3-methylimidazolium-bis(trifluormethansulfonimid), 1-Ethyl-3-methylimidazolium-thiocyanat, 1-Butyl-3-methylimidazolium-thiocyanat, 1-Ethyl-3-methylimidazolium-tetrafluorborat, N-(n-Hexyl)pyridinium-tetrafluorborat, N-(n-Hexyl)pyridinium-bis(trifluormethansulfonimid), N-(n-Butyl)-3-methylpyridinium-tetrafluorborat, N-(n-Butyl)-4-methylpydirdinium-tetrafluorborat, N-Methylpyrrolidin-Zink-Borhydrid, 1-Allyl-3-n-butyl-imidazolium-borhydrid, 1,3-Diallylimidazolium-borhydrid, 1,3-Di-(n-octyl)-imidazolium-borhydrid und 1,3-Di-(n-butyl)-imidazolium-borhydrid umfasst.

6. Geruchsprobe nach einem der vorhergehenden Ansprüche,
**dadurch gekennzeichnet,**
**dass** die ionische Flüssigkeit selbst gegenüber dem Sprengstoff reduzierend wirkt oder ein den Sprengstoff reduzierendes Reduktionsmittel enthält, wobei das Reduktionsmittel ein Zucker, ein Sulfit, Dithionit, Thiosulfat, Hydrazin, Boran, Phosphin, ein Hydrid, ein Metallhydrid, Zink, Magnesium, ein Siloxan oder ein Silan ist.

7. Geruchsprobe nach Anspruch 6,
**dadurch gekennzeichnet,**
**dass** der Zucker Glukose, Fructose, Galactose, Maltose oder Lactose und das Metallhydrid Lithiumaluminiumhydrid oder Borhydrid ist.

8. Geruchsprobe nach einem der Ansprüche 6 oder 7,
**dadurch gekennzeichnet,**
**dass** die selbst gegenüber dem Sprengstoff reduzierend wirkende ionische Flüssigkeit ein Thiocyanat, insbesondere 1-Ethyl-3-methylimidazolium-thiocyanat oder 1-Butyl-3-methylimidazolium-thiocyanat, oder ein Borhydrid, insbesondere N-Methylpyrrolidin-Zink-Borhydrid, 1-Allyl-3-n-butyl-imidazolium-borhydrid, 1,3-Diallylimidazolium-borhydrid, 1,3-Di-(n-octyl)-imidazolium-borhydrid oder 1,3-Di-(n-butyl)-imidazolium-borhydrid, ist.

9. Geruchsprobe nach einem der vorhergehenden Ansprüche,
**dadurch gekennzeichnet,**
**dass** der Sprengstoff in der ionischen Flüssigkeit in einer Konzentration von höchstens 12,5 Gew.-% oder höchstens 10 Gew.-% vorliegt.

10. Geruchsprobe nach einem der vorhergehenden Ansprüche,
**dadurch gekennzeichnet,**
**dass** der Sprengstoff in der ionischen Flüssigkeit in einer Konzentration von mindestens 1 Gew.-%, mindestens 2,5 Gew.-%, mindestens 5 Gew.-%, mindestens 7,5 Gew.-% oder mindestens 9 Gew.-% vorliegt.

11. Verfahren zur Herstellung der Geruchsprobe nach einem der vorhergehenden Ansprüche,
**dadurch gekennzeichnet,**
**dass** der nicht-peroxidische Sprengstoff in der ionischen Flüssigkeit aufgelöst wird.

12. Verwendung der Geruchsprobe nach einem der Ansprüche 1 bis 10 als Duftquelle, insbesondere für Testmessungen für die Kalibrierung von Detektoren und zum Training von Sprengstoffspürhunden oder von anderen zum Aufspüren von Sprengstoff geeigneten Tieren, insbesondere Ratten.

## Claims

1. Odour sample for explosives detection dogs, comprising a solution of an explosive in an ionic liquid, wherein the explosive is a non-peroxidic explosive, and wherein the explosive is present in the ionic liquid in a concentration of not more than 15% by weight.

2. Odour sample according to Claim 1,
**characterized in that**
the nonperoxidic explosive is hexogen (RDX), octogen (HMX), nitropenta (PETN), tetryl or trinitrotoluene (TNT).

3. Odour sample according to any of the preceding claims,
**characterized in that**
the ionic liquid contains anions selected from a group comprising tetrafluoroborates, triflimides, perfluoroalkylsulphates, alkylsulphonates, dicyandiamides, alkylsulphates, arylsulphonates, perfluoroalkylsulphonates, bis-perfluoroalkylsulphonimides, acetates, alkylcarboxylates, thiocyanates, isocyanates, isothiocyanates, thiosulphates, borohydrides, borates, phosphates, nitrates, perchlorates and halides, in particular iodides, bromides, chlorides and fluorides.

4. Odour sample according to any of the preceding claims,
**characterized in that**
the ionic liquid contains cations selected from a group comprising N-alkyl-substituted nitrogen heterocycle ions, in particular N-alkylpyridinium, N-alkylimidazolium and N,N-dialkylimidazolium ions, quaternary ammonium ions and phosphonium ions.

5. Odour sample according to any of the preceding claims,
**characterized in that**
the ionic liquid is selected from a group comprising
1-ethyl-3-methylimidazolium ethylsulphate, 1-ethyl-3-methylimidazolium-bis(trifluoromethanesulphonamide), 1-butyl-3-methylimidazolium-bis(trifluoromethanesulphonimide), 1-hexyl-3-methylimidazolium-bis(trifluoromethanesulphonimide), 1-ethyl-3-methylimidazolium thiocyanate, 1-butyl-3-methylimidazolium thiocyanate, 1-ethyl-3-methylimidazolium tetrafluoroborate, N-(n-hexyl)pyridinium tetrafluoroborate, N-(n-hexyl)pyridinium-bis(trifluoromethanesulphonimide), N-(n-butyl)-3-methylpyridinium tetrafluoroborate, N-(n-butyl)-4-methylpyridinium tetrafluoroborate, N-methylpyrrolidine-zinc borohydride, 1-allyl-3-n-butylimidazolium borohydride, 1,3-diallylimidazolium borohydride, 1,3-di(n-octyl)imidazolium borohydride and 1,3-di(n-butyl)imidazolium borohydride.

6. Odour sample according to any of the preceding claims,
**characterized in that**
the ionic liquid itself has a reducing action in respect of the explosive or contains a reducing agent which reduces the explosive, wherein the reducing agent is a sugar, a sulphite, dithionite, thiosulphate, hydrazine, borane, phosphine, a hydride, a metal hydride, zinc, magnesium, a siloxane or a silane.

7. Odour sample according to Claim 6,
**characterized in that**
the sugar is glucose, fructose, galactose, maltose or lactose and the metal hydride is lithium aluminium hydride or borohydride.

8. Odour sample according to either of Claims 6 and 7,
**characterized in that**
the ionic liquid which itself has a reducing action in respect of the explosive is a thiocyanate, in particular 1-ethyl-3-methylimidazolium thiocyanate or 1-butyl-3-methylimidazolium thiocyanate, or a borohydride, in particular N-methylpyrrolidine-zinc borohydride, 1-allyl-3-n-butylimidazolium borohydride, 1,3-diallylimidazolium borohydride, 1,3-di(n-octyl)imidazolium borohydride or 1,3-di(n-butyl)imidazolium borohydride.

9. Odour sample according to any of the preceding claims,
**characterized in that**
the explosive is present in a concentration of not more than 12.5% by weight or not more than 10% by weight in the ionic liquid.

10. Odour sample according to any of the preceding claims,
**characterized in that**
the explosive is present in a concentration of at least 1% by weight, at least 2.5% by weight, at least 5% by weight, at least 7.5% by weight or at least 9% by weight in the ionic liquid.

11. Process for producing the odour sample according to any of the preceding claims, **characterized in that** the nonperoxidic explosive is dissolved in the ionic liquid.

12. Use of the odour sample according to any of Claims 1 to 10 as scent source, in particular for test measurements for calibrating detectors and for the training of explosives detection dogs or of other animals suitable for detecting explosive, in particular rats.

## Revendications

1. Échantillon odorant pour chiens détecteurs d'explosifs, comprenant une solution d'un explosif dans un liquide ionique,
l'explosif étant un explosif non de type peroxyde, et l'explosif étant présent dans le liquide ionique à une concentration d'au maximum 15 % en poids.

2. Échantillon odorant selon la revendication 1,
**caractérisé en ce que**
l'explosif non de type peroxyde est l'hexogène (RDX), l'octogène (HMX), le nitropenta (PETN), le tétryl ou le trinitrotoluène (TNT).

3. Échantillon odorant selon l'une quelconque des revendications précédentes,
**caractérisé en ce que**
le liquide ionique contient des anions qui sont choisis dans un groupe qui comprend les tétrafluoroborates, triflimides, perfluoroalkylsulfates, alkylsulfonates, dicyanodiamides, alkylsulfates, arylsulfonates, perfluoroalkylsulfonates, bis-perfluoroalkyl-sulfonimides, acétates, alkylcarboxylates, thiocyanates, isocyanates, isothiocyanates, thiosulfates, borohydrures, borates, phosphates, nitrates, perchlorates et halogénures, en particulier iodures, bromures, chlorures et fluorures.

4. Échantillon odorant selon l'une quelconque des revendications précédentes,
**caractérisé en ce que**
le liquide ionique contient des cations qui sont choisis dans un groupe qui comprend des ions hétérocycliques azotés substitués par N-alkyle, en particulier des ions N-alkylpyridinium, N-alkylimidazolium et N,N-dialkylimidazolium, des ions phosphonium et ammonium quaternaires.

5. Échantillon odorant selon l'une quelconque des revendications précédentes,
**caractérisé en ce que**
le liquide ionique est choisi dans le groupe qui contient
de l'éthylsulfate de 1-éthyl-3-méthyl-imidazolium, du 1-éthyl-3-méthylimidazolium-bis(trifluorométhane-sulfonamide), du 1-butyl-3-méthylimidazolium-bis(trifluorométhane-sulfonimide), du 1-hexyl-3-méthylimidazolium-bis(trifluorométhane-sulfonimide), du thiocyanate de 1-éthyl-3-méthylimidazolium, du thiocyanate de 1-butyl-3-méthylimidazolium, du tétrafluoroborate de 1-éthyl-3-méthylimidazolium, du tétrafluoroborate de N-(n-hexyl)pyridinium, du N-(n-hexyl)pyridinium-bis(trifluorométhane-sulfonimide), du tétrafluoroborate de N-(n-butyl)-3-méthylpyridinium, du tétrafluoroborate de N-(n-butyl)-4-méthylpyridinium, du borohydrure de N-méthylpyrrolidine-zinc, du borohydrure de 1-allyl-3-n-butyl-imidazolium, du borohydrure de 1,3-diallylimidazolium, du borohydrure de 1,3-di-(n-octyl)-imidazolium et du borohydrure de 1,3-di-(n-butyl)-imidazolium.

6. Échantillon odorant selon l'une quelconque des revendications précédentes,
**caractérisé en ce que**
le liquide ionique a lui-même un effet réducteur vis-à-vis de l'explosif ou contient un réducteur réduisant l'explosif, le réducteur étant un sucre, un sulfite, dithionite, thiosulfate, une hydrazine, un borane, une phosphine, un hydrure, un hydrure métallique, du zinc, du magnésium, un siloxane ou un silane.

7. Échantillon odorant selon la revendication 6,
**caractérisé en ce que**
le sucre est le glucose, le fructose, le galactose, le maltose ou le lactose et l'hydrure métallique est l'hydrure de lithium et d'aluminium ou l'hydrure de bore.

8. Échantillon odorant selon l'une quelconque des revendications 6 et 7,
**caractérisé en ce que**
le liquide ionique ayant lui-même un effet réducteur vis-à-vis de l'explosif est un thiocyanate, en particulier le thiocyanate de 1-éthyl-3-méthyl-imidazolium ou le thiocyanate de 1-butyl-3-méthyl-imidazolium, ou un borohydrure, en particulier le borohydrure de N-méthylpyrrolidine-zinc, le borohydrure de 1-allyl-3-n-butyl-imidazolium, le borohydrure de 1,3-diallylimidazolium, le borohydrure de 1,3-di-(n-octyl)-imidazolium ou le borohydrure de 1,3-di-(n-butyl)-imidazolium.

9. Échantillon odorant selon l'une quelconque des revendications précédentes,
**caractérisé en ce que**
l'explosif est présent dans le liquide ionique à une concentration d'au maximum 12,5 % en poids ou d'au maximum 10 % en poids.

10. Échantillon odorant selon l'une quelconque des revendications précédentes,
**caractérisé en ce que**
l'explosif est présent dans le liquide ionique à une concentration d'au moins 1 % en poids, d'au moins 2,5 % en poids, d'au moins 5 % en poids, d'au moins 7,5 % en poids ou d'au moins 9 % en poids.

11. Procédé pour la préparation de l'échantillon odorant selon l'une quelconque des revendications précédentes,
**caractérisé en ce que**
l'explosif non de type peroxyde est dissous dans le liquide ionique.

12. Utilisation de l'échantillon odorant selon l'une quelconque des revendications 1 à 10 en tant que source d'odeur, en particulier pour des mesures d'essai pour l'étalonnage de détecteurs et pour l'apprentissage de chiens détecteurs d'explosifs ou d'autres animaux appropriés à la détection d'explosifs, en particulier de rats.
